(19) Europäisches Patentamt
European Patent Office
Office européen des brevets

(11) Publication number: **0 497 163 B1**

(12) # EUROPEAN PATENT SPECIFICATION

(45) Date of publication of patent specification: **07.12.94**

(51) Int. Cl.5: **A61K 7/06**

(21) Application number: **92100733.2**

(22) Date of filing: **17.01.92**

(54) **Shampoo composition.**

(30) Priority: **30.01.91 JP 29597/91**

(43) Date of publication of application:
**05.08.92 Bulletin 92/32**

(45) Publication of the grant of the patent:
**07.12.94 Bulletin 94/49**

(84) Designated Contracting States:
**AT CH DE GB LI NL**

(56) References cited:
**EP-A- 0 117 360**
**EP-A- 0 285 388**
**EP-A- 0 312 234**
**EP-A- 0 412 710**

(73) Proprietor: **KAO CORPORATION**
**14-10, Nihonbashi Kayaba-cho 1-chome**
**Chuo-ku Tokyo (JP)**

(72) Inventor: **Kobayashi, Hisataka**
**3-9-15 Higashisyukugou**
**Utsunomiya-shi, Tochigi (JP)**
Inventor: **Kamegai, Jun**
**4-2-55-506 Shiohama**
**Ichikawa-shi, Chiba (JP)**

(74) Representative: **Kraus, Walter, Dr. et al**
**Patentanwälte Kraus, Weisert & Partner**
**Thomas-Wimmer-Ring 15**
**D-80539 München (DE)**

**EP 0 497 163 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to a shampoo composition, and more particularly, to a shampoo composition containing a modified silicone polymer compound and a pyridinethione salt. The shampoo composition provides an excellent anti-dandruff effect, an excellent soft and smooth feeling and no creakiness during washing, rinsing or after drying the hair.

Pyridinethione salts have an excellent antidandruff effect and have been used as an ingredient of shampoo. A shampoo containing a pyridinethione salt, however, tends to give a creaky feeling during washing and rinsing.

Hair care is increasingly more important, and a creaky feeling of the hair during washing and rinsing suggests to people that the hair is being damaged. Therefore, reducing the creakiness of the hair when using a shampoo composition containing a pyridinethione salt is an important objective of the field.

On the other hand, although surface active agents in shampoo compositions also give a creaky feeling to the hair during washing and rinsing, creakiness is reduced by adding a cationic polymer to the shampoo composition. Addition of a cationic polymer to a shampoo composition may also be used to reduce the creakiness of the hair caused by a pyridinethione salt. A large quantity of cationic polymer, however, must be added to the composition in order to reduce the creakiness caused by the pyridinethione salt. A shampoo composition containing a large quantity of cationic polymer gives not only a sticky feeling during drying of the hair, but also a stiffness to the hair because of the chemical complex of surface active agent and cationic polymer.

Shampoo compositions containing a pyridinethione salt and an insoluble silicone are disclosed in Japanese Laid Open Patent Application Nos. 64-13012/1989, 64-13013/1989, and European Patent Publication No. 285388. The shampoo compositions of these patent applications give conditioning effects to dry hair but don't have the effect of reducing creakiness of the hair during washing and rinsing of the hair.

It is desirable, therefore, to provide a shampoo composition which has an excellent antidandruff effect and gives no creaky feeling to the hair during washing and rinsing, and gives no stickiness and stiffness after drying of the hair.

Accordingly, it is an object of this invention to provide a shampoo composition containing a pyridinethione salt which has an excellent antidandruff effect and gives no creaky feeling to the hair during washing and rinsing, and gives no stickiness and stiffness after drying of the hair.

This and other objects of the invention as will become more apparent by the following description have been achieved by the use of a specific type of modified silicone polymer compound in combination with a pyridinethione salt. The resulting shampoo composition is found to give no creaky feeling during washing and rinsing, no stickiness and stiffness after drying of the hair, and in addition, a natural hair-setup performance, good comb passage, and an improved antidandruff effect.

A shampoo composition has been discovered which comprises:

(a) one or more surface active agents selected from the group consisting of anionic surfactants, amphoteric surfactants, and nonionic surfactants,

(b) a modified silicone polymer compound containing in a molecule thereof at least one aminoalkyl group and at least one group selected from hydroxyl, hydroxyalkyl, oxyalkyl, and polyoxyalkylene groups, and

(c) a pyridinethione salt.

The following known surface active agents may be used as component (a) of the invention. Suitable anionic surface active agents include

(1) alkyl benzenesulfonates having $C_{10-16}$ linear or branched alkyl or alkenyl groups,

(2) alkyl or alkenyl ether sulphates having in a molecule thereof a $C_{10-20}$ linear or branched alkyl or alkenyl group, and 0.5 to 8 moles of alkylene oxide groups selected from the group consisting of ethylene oxide, propylene oxide, butylene oxide, mixtures of ethylene oxide and propylene oxide having a molar ratio of 0.1/9.9 to 9.9/0.1, and mixtures of ethylene oxide and butylene oxide having a molar ratio of 0.1/9.9 to 9.9/0.1,

(3) alkyl or alkenyl sulphates having $C_{10-20}$ alkyl or alkenyl groups,

(4) olefin sulfonates having 10 to 20 carbon atoms,

(5) alkane sulfonates having 10 to 20 carbon atoms,

(6) saturated or unsaturated fatty acids having 10 to 24 carbon atoms,

(7) alkyl or alkenyl ether carbonates having $C_{10-20}$ linear or branched alkyl or alkenyl groups, and 0.5 to 8 moles of alkylene oxide groups selected from the group consisting of ethylene oxide, propylene oxide, butylene oxide, mixtures of ethylene oxide and propylene oxide having a molar ratio of 0.1/9.9 to 9.9/0.1, and mixtures of ethylene oxide and butylene oxide having a molar ratio of 0.1/9.9 to 9.9/0.1,

(8) alpha-sulfo fatty acid esters or salts having 10 to 20 carbon atoms,

(9) N-acyl amino acid surface active agents having $C_{8-24}$ acyl groups and a carboxyl group,
(10) phosphoric acid mono- or diester surface active agents having $C_{8-24}$ alkyl or alkenyl groups, and
(11) polyoxyether alkyl sulfo succinic acid monoester salts having $C_{8-16}$ alkyl groups.

Suitable amphoteric surface active agents include
(12) alpha-substituted secondary amide or tertiary amide imidazoline amphoteric surface active agents having $C_{8-24}$ alkyl or alkenyl groups, and
(13) carbobetaine, amidobetaine, sulfobetaine, hydroxy sulfobetaine, or amidosulfobetaine amphoteric surface active agents having $C_{8-24}$ alkyl or alkenyl groups.

Suitable nonionic surface active agents include
(14) higher fatty acid alkyl amides having $C_{10-20}$ long chain acyl groups or the product thereof derived from condensation with ethylene oxide, and
(15) alkyl saccharide surface active agents represented by the following formula

$R^1$-O-($R^2$O)$_m$-(G)$_n$ (1)

where,
$R^1$: $C_{6-18}$ linear or branched alkyl or alkenyl group,
$R^2$: $C_{2-4}$ alkyl group,
G: $C_{5-6}$ reducing sugar,
m: 0 to 10, and
n: 1 to 10.

The surface active agents are incorporated into the composition of the present invention as component (a) either independently or in combination, in an amount of 5 to 30% by weight, and preferably 10 to 20% by weight.

Modified silicone polymer compounds of any molecular structure, e.g. branched, linear, and netting types, can be used as component (b) so long as they contain at least one aminoalkyl group and at least one hydroxyl, hydroxylalkyl, oxyalkylene, or polyoxyalkylene group. Organopolysiloxanes which are used to synthesize the modified silicone polymer compounds may include, in addition to the groups noted above, alkyl groups, e.g. methyl, ethyl, propyl; alkenyl groups, e.g. allyl, vinyl; aryl groups, e.g. phenyl, naphthyl; cycloalkyl groups, e.g. cyclohexyl; and the like. Compounds containing methyl group are preferably used. Modified silicone polymer compounds which may be used in the present invention are commercially available.

Typical aminoalkyl groups on the modified silicone polymer compound are shown below.

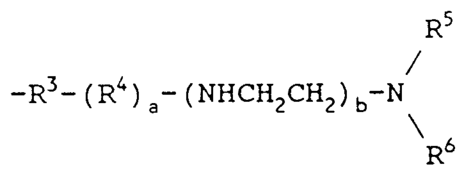
(2)

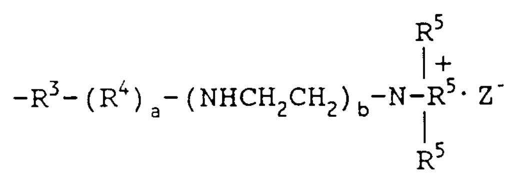
(2')

where $R^3$ represents a divalent hydrocarbon group, $R^4$ represents a group, $-OCH_2CH_2-$,

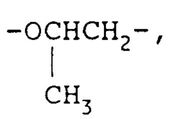 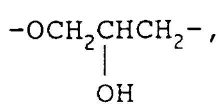 or 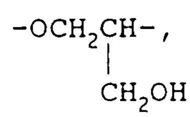

where $R^5$ and $R^6$ are, individually, a hydrogen or a monovalent hydrocarbon group, and a and b denote integers of 1-6, $Z^-$ represents a halogen ion or an organic anion. As divalent hydrocarbon groups

represented by $R^3$, alkylene groups, e.g. methylene, ethylene, propylene, butylene, $-CH_2CH(CH_3)CH_2-$; and alkylene-arylene groups, e.g. $-(CH_2)_2-C_6H_4-$, are preferred. Of these groups, alkylene groups, particularly the propylene group, are most prefered. Alkyl groups, e.g. methyl, ethyl, propyl, hexyl, and the phenyl group are prefered monovalent hydrocarbon groups represented by $R^5$ and $R^6$. Both $R^5$ and $R^6$ may be a hydrogen atom, or both may be monovalent hydrocarbon groups, or either one of $R^5$ and $R^6$ is a hydrogen, with the other being a monovalent hydrocarbon group. A preferable value for a and b are $a = 0$, $b = 1$.

A typical hydroxyalkyl group on the modified silicone polymer compound has the following formula (3)

$-R^3-OH$ (3)

where $R^3$ has the same meaning as defined above.

Oxyalkylene and polyoxyalkylene groups are typified by the groups having following formula (4)

$-(R^3)_c-O-(C_eH_{2e}O)_d-H$ (4)

where $R^3$ has the same meaning as defined above, c represents 0 or 1, d denotes an integer of 1 to 100, and e denotes an integer of 1 to 5.

Of the hydroxyalkyl groups represented by formula (4), those in which $c = 1$, $d = 3$ to 70, and $e = 2$ or 3 are preferable. A hydroxyalkyl group produced by random or block polymerization of a group where the value $e = 2$ and a group where the value $e = 3$ is preferred. Random and block polymerization may also be used to prepare hydroxyalkyl groups when e is other than 2 or 3.

Typical modified silicone polymer compounds are those represented by formulae (5) and (6).

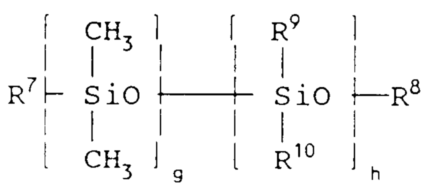

(5)

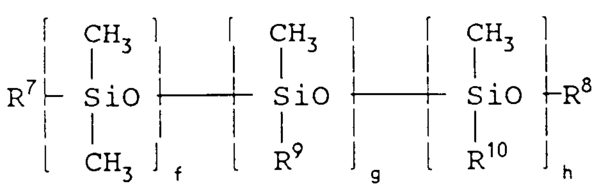

(6)

where $R^7$ is a methyl or hydroxy group, and $R^8$ is a methyl group or a hydrogen, $R^9$ is the above-mentioned aminoalkyl group (2) or (2'), $R^{10}$ is a hydroxy, hydroxyalkyl, oxyalkylene, or polyoxyalkylene group, and f, g, and h are integers dependent on the molecular weight. Modified silicone polymer compounds represented by formula (7) are particularly preferred.

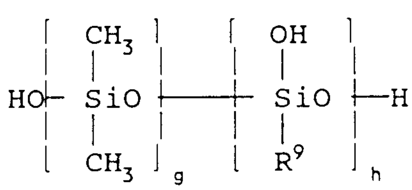

(7)

where $R^9$ is the above-mentioned aminoalkyl group (2) or (2'), and g, h are integers dependent on the molecular weight.

The modified silicone polymers having formulas (5), (6) and (7) generally have average molecular weights ranging from about 300 to 1,000,000, preferably 3,000 to about 100,000. Subscripts f, g and h can be readily determined for any particular polymer having a specific average molecular weight. The average

molecular weight can be determined according to conventional methods.

One specific example of the modified silicone polymer compound is that described in Cosmetic Ingredient Dictionary, third edition, having the name of Amodimethicone. This compound is represented by formula (8) and has an average molecular weight of 3,000 to 100,000.

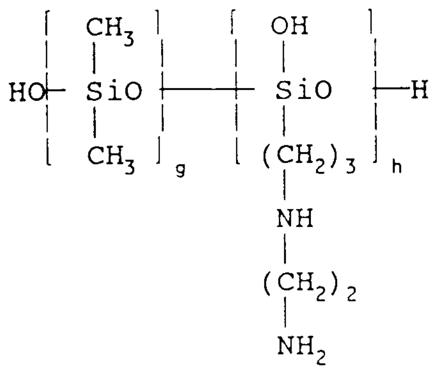

(8)

where g and h are integers dependent on the molecular weight.

It is desirable that the modified silicone polymer compounds of the present invention be used in the form of an aqueous emulsion. Such an emulsion can be obtained by emulsifying, in the presence of a quaternary ammonium salt surfactant and water, a cyclic diorganopolysiloxane and an organodialkoxysilane having an aminoalkyl group and at least one hydroxyl, hydroxyalkyl, oxyalkylene, and polyoxyalkylene group, according to the process described, for example, in Japanese Laid-Open Patent Application No. 56-38609/1981.

When the modified silicone polymer compound is used in the form of an aqueous emulsion, the amount of modified silicone polymer compounds in the emulsion is usually 20 to 60% by weight, and preferably 30 to 50 % by weight.

Examples of commercially available modified silicone polymer emulsions which can be suitably used in this invention are SM8702C (Tradename, product of Toray Silicone Co.) and DC929 (Tradename, product of Dow Corning Co.).

Compound (b) is used in an amount of 0.005-5% by weight, and preferably 0.01-2% by weight, in the composition of the present invention. When component (b) is in the form of an aqueous emulsion, the amount of the aqueous emulsion incorporated into the composition of the present invention is 0.01-8% by weight, and preferably 0.05-3% by weight.

Suitable pyridinethione salts (c) for use in the present composition include polyvalent metal salts of the pyridinethione represented by formula (9)

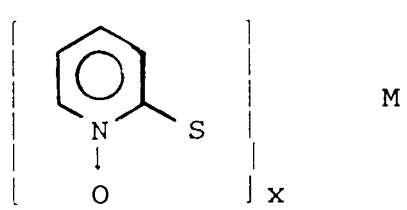

(9)

where M represents a polyvalent metal atom, and x denotes the valence of M. Suitable polyvalent metals include magnesium, barium, strontium, zinc, cadmium, tin and zirconium, and preferably zinc.

Component (c) is used in an amount of 0.1 to 5% by weight, preferably 0.5 to 2% by weight. If the amount of component (c) is less than 0.1% by weight, the antidandruff effect is not achieved. However, the antidandruff effect does not increase even if the amount of component (c) is more than 5% by weight.

Besides the three essential components, various components commonly known for use in a shampoo composition can be formulated to the extent that the effects of the present invention are not adversely affected. Such optional components include, for example, a suspension agent, e.g. ethyleneglycol distearate, ethyleneglycol monostearate; a dissolving agent, e.g. propyleneglycol, glycerin; a viscosity adjusting agent, e.g. ethanol, inorganic salt, higher alcohol, hydroxymethyl cellulose, hydroxyethyl cellulose, hydroxypropyl cellulose, methyl cellulose; conditioning agents, e.g. cationic polymers; perfumes, coloring agents, UV absorbers, antioxidants, antiseptics, and pH adjustment agents.

The shampoo composition of the present invention can be prepared in liquid, paste or any other forms, according to conventional methods by using components (a), (b) and (c).

The composition gives exceptionally good feeling without creakiness during washing and rinsing and softly finished smooth hair after drying as well as even better hair-setup performance, and furthermore, exhibits a high antidandruff effect.

## EXAMPLES

The feeling of creakiness, passage of fingers through the hair, hair-setup performance, and antidandruff effect were evaluated as follows.

### Creakiness

In the evaluation, 1g of sample composition was applied to a bundle of hair (weight; 20g, length; 15cm) of a healthy Japanese woman. Feeling to the touch was determined after the shampoo was foamed for 1 minute and rinsed for 30 seconds. Creakiness of the hair was evaluated by 5 expert panelists according to the following standards.

〈Evaluation Standard〉

Creakiness:

A: Hair gives no creaky feeling at all,
B: Hair gives only slight creaky feeling,
C: Hair gives rather strong creaky feeling.

### Hair-setup performance and passage of fingers through the hair

In the evaluation, 1 g of sample composition was applied to a bundle of hair (weight-20g, length-15cm) of a healthy Japanese woman. Feeling to the touch was determined after the shampoo was foamed for 1 minute, rinsed for 30 seconds, then dried with a towel and further dried with a hot air dryer. Hair-setup performance and passage of fingers through the hair were evaluated by 5 expert panelists according to the following standards.

〈Evaluation standard of Hair-setup performance〉

A: Hair is naturally set up,
B: Set-up is not always complete,
C: Set-up is incomplete with many hair wisp or strands.

〈Evaluation standard of passage of fingers through the hair〉

A: Hair gives no creaky feeling, fingers pass through very easily,
B: Hair gives only slight creaky feeling, fingers pass through easily,
C: Hair gives strong creaky feeling, fingers do not pass through easily.

### Antidandruff effect

Five males age 20 to 30 having dandruff were selected as testees. They could not improve their dandruff hair by using a conventional shampoo.

The antidandruff effect was evaluated as follows. The testees' hair was washed once a day for two weeks using a conventional shampoo containing 20% by weight of lauryl sulfate triethanolamine salt, and the appearance of dandruff was then observed by the naked eye as the control data. Their hair was then washed once a day for a month using the shampoo composition of the present invention. The appearance of dandruff after a month was observed by the naked eye.

Comparing the appearance of dandruff of the control period with that of this invention, the antidandruff effect was evaluated by the following evaluation standard.

⟨Evaluation standard of Antidandruff effect⟩

A: High antidandruff effect, quantity of dandruff was less than 25% of the control period,
B: Medium antidandruff effect, quantity of dandruff was between 25 and 50% of the control period,
C: Low antidandruff effect, quantity of dandruff was between 50 and 75% of the control period,
D: No antidandruff effect, quantity of dandruff was between 75 and 100% of the control period.

Example 1

Shampoo compositions were prepared according to the formulations listed in Table 1. Each composition was tested in terms of its feeling (sensory evaluation) during washing, rinsing, after drying, and its antidandruff effect.

Obviously, numerous modifications and variations of the present invention are possible in light of the above teachings. It is therefore to be understood that within the scope of the appended claims, the invention may be practiced otherwise than as specifically described herein.

Table 1

| Components (% by weight) | Comparative compositions 1 | 2 | 3 | 4 | 5 | 6 | Invention compositions 7 | 8 | 9 |
|---|---|---|---|---|---|---|---|---|---|
| Lauryl sulfate triethanolamine | 20 | | | 20 | | | 20 | | |
| Sodium polyoxyethylene lauryl ether sulfate | | 20 | | | 20 | | | 20 | |
| Decyl polyglucoside (degree of polymerization: 1.4) | | | 20 | | | 20 | | | 20 |
| Lauric acid diethanolamide | 3 | 3 | 3 | 3 | 3 | 3 | 3 | 3 | 3 |
| Zinc pyrithion | 1 | 1 | 1 | 1 | 1 | 1 | 1 | 1 | 1 |
| Amodimethicone emulsion* | | | | | | | 0.1 | 0.1 | 0.1 |
| Dimethylpolysiloxane (100,000 cs) | | | | 1 | 1 | 1 | | | |
| Cationic polymer (Polymer JR-400, product of UCC) | | | | | | | | | |
| Water (balance) | | | | | | | | | |
| Creakiness | C | C | C | C | C | C | A | A | A |
| Passage of fingers through the hair | B | B | C | A | A | A-B | A | A | A |
| Hair-setup performance | C | C | B | B | B | A | A | A | A |
| Antidandruff effect | B | B | A-B | B | B | A-B | A-B | A-B | A |

*Modified silicone polymer emulsion SM8702C (comprising 40% by weight of amodimethicone, product of Toray Silicone Co.)

## Claims

**1.** A shampoo composition comprising:

(a) a surface active agent selected from the group consisting of anionic surfactants, amphoteric surfactants, and nonionic surfactants,

(b) a modified silicone polymer containing in a molecule thereof at least one aminoalkyl group and at least one group selected from the group consisting of hydroxyl, hydroxyalkyl, oxyalkylene, and polyoxyalkylene groups, and

(c) a pyridinethione salt.

2. The shampoo composition according claim 1, wherein said surface active agent is present in an amount of 5-30% by weight.

3. The shampoo composition according claim 1, wherein said surface active agent is present in an amount of 10-20% by weight.

4. The shampoo composition according claim 1, wherein said silicone polymer is present in an amount of 0.005-5% by weight.

5. The shampoo composition according claim 1, wherein said silicone polymer is present in an amount of 0.01-2% by weight.

6. The shampoo composition according claim 1, wherein said pyridinethione salt is present in an amount of 0.1-5% by weight.

7. The shampoo composition according claim 1, wherein said pyridinethione salt is present in an amount of 0.5-2% by weight.

8. The shampoo composition according claim 1, wherein said pyridinethione salt is a polyvalent metal salt of pyridinethione.

9. The shampoo composition according claim 8, wherein said polyvalent metal is selected from the group consisting of magnesium, calcium, strontium, zinc, cadmium, tin, and zirconium.

10. The shampoo composition according claim 8, wherein said polyvalent metal is zinc.

**Patentansprüche**

1. Shampoo bzw. Haarwaschmittel, dadurch **gekennzeichnet,** daß es
   (a) ein grenzflächenaktives Mittel, ausgewählt aus der Gruppe bestehend aus anionischen Tensiden, amphoteren Tensiden und nichtionischen Tensiden,
   (b) ein modifiziertes Siliconpolymeres, das im Molekül mindestens eine Aminoalkylgruppe und mindestens eine Gruppe, ausgewählt aus der Gruppe bestehend aus Hydroxyl-, Hydroxyalkyl-, Oxyalkylen- und Polyoxyalkylengruppen enthält und
   (c) ein Pyridinthionsalz
   enthält.

2. Shampoo nach Anspruch 1, dadurch **gekennzeichnet,** daß das grenzflächenaktive Mittel in einer Menge von 5 bis 30 Gew.-% vorliegt.

3. Shampoo nach Anspruch 1, dadurch **gekennzeichnet,** daß das grenzflächenaktive Mittel in einer Menge von 10 bis 20 Gew.-% vorliegt.

4. Shampoo nach Anspruch 1, dadurch **gekennzeichnet,** daß das Siliconpolymere in einer Menge von 0,005 bis 5 Gew.-% vorliegt.

5. Shampoo nach Anspruch 1, dadurch **gekennzeichnet,** daß das Siliconpolymere in einer Menge von 0,01 bis 2 Gew.-% vorliegt.

6. Shampoo nach Anspruch 1, dadurch **gekennzeichnet,** daß das Pyridinthionsalz in einer Menge von 0,1 bis 5 Gew.-% vorliegt.

7. Shampoo nach Anspruch 1, dadurch **gekennzeichnet,** daß das Pyridinthionsalz in einer Menge von 0,5 bis 2 Gew.-% vorliegt.

8. Shampoo nach Anspruch 1, dadurch **gekennzeichnet,** daß das Pyridinthionsalz ein mehrwertiges Metallsalz von Pyridinthion ist.

9. Shampoo nach Anspruch 8, dadurch **gekennzeichnet,** daß das mehrwertige Metall aus der Gruppe bestehend aus Magnesium, Calcium, Strontium, Zink, Cadmium, Zinn und Zirconium ausgewählt ist.

10. Shampoo nach Anspruch 8, dadurch **gekennzeichnet,** daß das mehrwertige Metall Zink ist.

**Revendications**

1. Composition de shampooing qui comprend :
   (a) un agent tensioactif choisi dans le groupe constitué des tensioactifs anioniques, des tensioactifs amphotères et des tensioactifs non ioniques,
   (b) un polymère de silicone modifié contenant dans sa molécule au moins un groupe aminoalkyle et au moins un groupe choisi dans le groupe constitué des groupes hydroxyle, hydroxyalkyle, oxyalkylène et polyoxyalkylène, et
   (c) un sel de pyridinethione.

2. Composition de shampooing selon la revendication 1, dans laquelle ledit agent tensioactif est présent en une quantité de 5-30 % en poids.

3. Composition de shampooing selon la revendication 1, dans laquelle ledit agent tensioactif est présent en une quantité de 10-20 % en poids.

4. Composition de shampooing selon la revendication 1, dans laquelle ledit polymère de silicone est présent en une quantité de 0,005-5 % en poids.

5. Composition de shampooing selon la revendication 1, dans laquelle ledit polymère de silicone est présent en une quantité de 0,01-2 % en poids.

6. Composition de shampooing selon la revendication 1, dans laquelle ledit sel de pyridinethione est présent en une quantité de 0,1-5 % en poids.

7. Composition de shampooing selon la revendication 1, dans laquelle ledit sel de pyridinethione est présent en une quantité de 0,5-2 % en poids.

8. Composition de shampooing selon la revendication 1, dans laquelle ledit sel de pyridinethione est un sel de métal polyvalent de pyridinethione.

9. Composition de shampooing selon la revendication 8, dans laquelle ledit métal polyvalent est choisi dans le groupe constitué du magnésium, du calcium, du strontium, du zinc, du cadmium, de l'étain et du zirconium.

10. Composition de shampooing selon la revendication 8, dans laquelle ledit métal polyvalent est le zinc.